# FASCICULE DE BREVET EUROPEEN

(11) **EP 1 301 192 B1**
(45) Date de publication et mention de la délivrance du brevet: **21.01.2009**
(21) Numéro de dépôt: 01954083.0
(22) Date de dépôt: 12.07.2001
(51) Int. Cl.: A61K 31/715, A61P 19/02

(54) **UTILISATION DE CYCLODEXTRINES POLYSULFATEES POUR LE TRAITEMENT DE L'ARTHROSE**
VERWENDUNG VON POLYSULFATIERTEN CYCLODEXTRINEN ZUR BEHANDLUNG VON ARTHROSE
USE OF POLYSULPHATED CYCLODEXTRINS FOR TREATING OSTEOARTHRITIS

(30) Priorité: 17.07.2000 FR 0009272
(43) Date de publication de la demande: 16.04.2003
(73) Titulaire: Universiteit Gent, 9000 Gent (BE)
(72) Inventeur: VEYS, Eric, Maximilien, B-9052 Gent (BE); VERBRUGGEN, August, Lodewijk, B-9810 Nazareth (BE)
(74) Mandataire: Bird, Ariane
(86) Numéro de dépôt international: PCT/FR2001/002253
(87) Numéro de publication internationale: WO 2002/005826

(56) Documents cités:
- EP-A- 0 447 171
- EP-A- 0 754 460
- WO-A-91/16905
- WO-A-92/13895
- WO-A-93/09790
- US-A- 4 247 535
- US-A- 4 258 180
- US-A- 5 843 920
- DA CAMARA: "Glucosamine sulfate for osteoarthrtis" ANNALS OF PHARMACOTHERAPY,XX,XX, vol. 32, no. 5, mai 1998 (1998-05), pages 580-587, XP002123880 ISSN: 1060-0280
- HAMERMAN D: "THE BIOLOGY OF OSTEOARTHRITIS" NEW ENGLAND JOURNAL OF MEDICINE, THE,US,MASSACHUSETTS MEDICAL SOCIETY, WALTHAM, MA, vol. 320, no. 20, 18 mai 1989 (1989-05-18), pages 1322-1330, XP000617789 ISSN: 0028-4793 cité dans la demande

## Description

La présente invention concerne l'utilisation d'une cyclodextrines polysulfatée ou une de ses sels d'addition comme seul ingredient actif pour l'obtention d'une composition pharmaceutique destinéé au traitement de l'arthrose.

L'utilisation en thérapeutique des cyclodextrines et de leurs dérivés synthétiques a été très largement étudiée au cours des quinze dernières années.

L'introduction des groupements sulfates sur les groupements hydroxy des cyclodextrines leur confère des propriétés biologiques particulièrement intéressantes. Il a notamment été montré que les cyclodextrines polysulfatées pourraient réduire ou bloquer les effets de substances tératogènes sur le développement du foetus (WO 9116905), qu'elles possédaient un pouvoir de cicatrisant tissulaire (WO 9309790). Elles sont également connues en tant qu'inhibiteurs de rétrovirus, HIV en particulier (EP 447 171) ou bien encore comme inhibiteurs d'angiogénèse (WO 8906536).

La Demanderesse a présentement découvert que, de façon surprenante, les cyclodextrines polysulfatées et leurs sels d'addition pouvaient être utilisées de façon avantageuse pour l'obtention de médicaments utiles dans le traitement de l'arthrose.

Les cyclodextrines polysulfatées utiles selon l'invention sont des α, β ou γ-cyclodextrines polysulfatées.

Parmi les sels d'addition des cyclodextrines polysulfatées, on peut citer les sels de sodium, de calcium, de potassium, d'ammonium.

L'arthrose se caractérise anatomiquement par une destruction initiale et primitive des cartilages articulaires.

En conditions normales, le renouvellement du cartilage est un processus très lent consistant en une phase de résorption par les chondrocytes, directement compensée par une phase de formation par ces mêmes chondrocytes.

En conditions pathologiques, le renouvellement du cartilage peut s'accélérer, conduisant à une réaction de réparation précoce du cartilage suivie d'une décompensation cellulaire et d'une dégradation du cartilage. La réaction de réparation résulte d'une multiplication clonale des chondrocytes et de leur synthèse accrue des composants matriciels du cartilage (D. Hamerman et coll., N. Engl. J. Med., 1989, 320(20), 1322-1330). Cette réaction homéostatique n'est pas adaptée et dépend d'hormones systémiques et de facteurs de croissance dont les sécrétions diminuent avec l'âge. La résorption du cartilage est régulée par des enzymes et des radicaux libres produits par des tissus adjacents, mais aussi et surtout par le chondrocyte lui-même.

En particulier, la résistance du cartilage et sa capacité à se réparer est déterminée par les protéoglycanes de la matrice extracellulaire, et notamment les aggrécanes. La synthèse de ces aggrécanes par les chondrocytes articulaires et leur qualité diminuent avec l'âge, ce qui constitue un des facteurs principaux impliqués dans le développement de l'arthrose.

Plus spécifiquement, il a été découvert que les cyclodextrines polysulfatées stimulent la synthèse des protéoglycanes et plus particulièrement de l'aggrécane et de ses agrégats par les cellules du cartilage articulaire humain.

Ceci a été confirmé par la mesure de l'incorporation du ³⁵S dans le réseau d'aggrécane par les chondrocytes et par l'augmentation des proportions d'aggrécanes avec pour conséquence une augmentation de la synthèse de l'aggrécane de la matrice pericellulaire.

L'utilisation en thérapeutique des cyclodextrines polysulfatées est d'autant plus intéressante que ces composés, à l'inverse d'autres polysaccharides, présentent des structures bien définies. Leur poids moléculaire est plus faible que celui de polysaccharides polysulfatés utilisés en thérapeutique comme le chondroïtine sulfate ou le chondroïtine polysulfate dont certains effets toxiques après administration parentérale ont été attribués à leur haut poids moléculaire.

D'autre part, les effets biologiques de ces cyclodextrines polysulfatées sont supérieurs à ceux des polysaccharides polysulfatées cités plus haut

Les cyclodextrines polysulfatées sont préparées par réaction de l'acide chlorosulfonique sur les cyclodextrines selon la méthode décrite par T. Astrup. et coll. (Acta Phys. Scand.,*1944*, 8,215-226).

Les compositions pharmaceutiques selon l'invention seront présentées sous des formes convenant pour l'administration par voie orale, parentérale, transcutanée, nasale, rectale, perlinguale et notamment sous forme de préparations injectables, comprimés, comprimés sublinguaux, glossettes, gélules, capsules, tablettes, suppositoires, crèmes, pommades, gels dermiques.

Les compositions selon l'invention contiennent, outre une quantité appropriée de cyclodextrine polysulfatée, un ou plusieurs excipients ou véhicules inertes pharmaceutiquement choisis notamment parmi les diluants, les lubrifiants,les liants, les agents de désintégration, les absorbants, les colorants, les édulcorants.

A titre d'exemple, on peut citer :
- *pour les diluants :* le lactose, le dextrose, le sucrose, le mannitol, le sorbitol, la cellulose, la glycérine,
- *pour les lubrifiants:* la silice, le talc, l'acide stéarique et ses sels de magnésium et de calcium, le polyéthylène glycol,
- *pour les liants* : le silicate d'aluminium et de magnésium, l'amidon, la gélatine, la tragacanthe, la méthylcellulose, la carboxyméthylcellulose et la polyvinyl-pyrrolidone,
- *pour les désintégrants* :l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 50 mg et 1500 mg en une à plusieurs prises par 24 heures.

### Préparation des cyclodextrines polysulfatées sous forme de sels de sodium

300 mg de cyclodextrine sont ajoutés à 5 ml d'une solution préparée par addition d'un volume d'acide chlorosulfonique dans 6,6 volumes de pyridine à 0°C. La solution ainsi obtenue est maintenue à 100°C pendant 5 jours. Après refroidissement, addition de 25 ml d'eau puis de 100 ml d'une solution à 10 % d'acétate de sodium dans le méthanol, le précipité formé est filtré et lavé par du méthanol. Le précipité est alors dissous dans de l'eau puis élué par chromatographie sur colonne de perméation de gel pour conduire à la cyclodextrine polysulfatée attendue qui est alors lyophilisée. Le degré de sulfatation de la cyclodextrine est déterminé par électrophorèse ou par spectrométrie de masse. Dans ces conditions opératoires, la sulfatation des cyclodextrines est complète.

### EXEMPLE 1:

### ETUDE PHARMACOLOGIQUE

Les études ont été réalisées avec les α, β et γ-cyclodextrines polysulfatées sous forme de sels de sodium.

Evaluation des effets in vitro des cyclodextrines polysulfatées sur la synthèse d'aggrécanes extracellulaires par les chondrocytes.

### Matériel et méthodes

Les chondrocytes articulaires humains sont isolés selon les méthodes décrites par W. T. Green Jr (Clin. Orthop., 1971, 75, 248 -260) et K. E. Kuettner & coll. (J. Cell. Biol., 1982, 93, 743-750).
Ces chondrocytes sont alors mis en culture dans du gel d'agarose selon la méthode décrite par P. D. Benya et coll. (Cell., 1982, 30, 215-224), et modifiée selon M. Comelissen et coll. (J. Tiss. Cult. Meth., 1993, 15 ,139 -146) et selon G. Verbruggen et coll. (Clin. Exp. Rheumatol,1990, 8, 371-378).

Synthèse d'aggrécanes : elle est mesurée par la détermination de l'incorporation du ³⁵S fourni par un précurseur radioactif, le sulfate de sodiumNa₂³⁵SO₄. Après deux semaines de culture, 10 µCi/ml du précurseur radiomarqué sont introduits dans le milieu de culture pendant 48 heures, ainsi que les composés à tester (α, β, γ cyclodextrines polysulfatées) ou les produits de référence (chondroïtine sulfate et polysulfates) à la concentration finale de 2,5µg/ml.
Les ³⁵S aggrécanes nouvellement synthétisés s'accumulent partiellement dans la matrice d'agarose intercellulaire ou sont libérés dans le milieu d'incubation.
En fin de période d'incubation, le gel d'agarose est cassé mécaniquement puis digéré par 3 ml d'une solution d'agarose à 50 U/ml dans un tampon phosphate 0,067 M à pH 6,0, en présence d'inhibiteurs de protéinase.
La suspension ainsi obtenue est centrifugée, le surnageant qui contient les ³⁵S aggrécanes de la matrice interterritoriale, et le milieu d'incubation contenant les métabolites de ³⁵S aggrécanes libérés dans la matrice extracellulaire sont réunis pour chromatographie ultérieure.

Le culot, contenant les chondrocytes et les ³⁵S aggrécanes qui leur sont associés, est traité pendant48 heures par 1 ml d'une solution de chlorure de guanidinium 4,0M dans un tampon acétate 0,05M à pH 5,8 contenant les inhibiteurs de protéinase.
Cette opération a pour but d'extraire les ³⁵S aggrécanes associés aux cellules. La solution obtenue est centrifugée pour séparer les cellules du surnageant qui est séparé pour chromatographie ultérieure. Les opérations de chromatographie des différentes fractions obtenues sont réalisées sur gel de Sephodex G25 dans un tampon phosphate pH 6,8 contenant 0,01M du Na₂SO₄, de manière à séparer les ³⁵S aggrécanes du Na₂³⁵SO₄ libre.
La radioactivité de chacun des éluats macromoléculaires obtenus est mesurée et rapportée au nombre de chondrocytes contenus dans la culture initiale et exprimée en pg de SO₄ incorporés dans les aggrécanes, par millions de chondrocytes et par heure.

### Résultats

Ils sont rassemblés dans le tableau ci-dessous.

| | **Quantité de ³⁵S aggrécanes** | | |
|---|---|---|---|
| **Produits testés* (2,5 µg/ml)** | **Associés aux cellules** | **de la matrice interterritoriale** | **dans le milieu** |
| α CD | 1315 | 8036 | 1589 |
| β CD | 2262 | 6232 | 1658 |
| γ CD | 2907 | 8232 | 3873 |
| CS | 1373 | 6589 | 2187 |
| CPS | 1392 | 6446 | 2519 |
| Contrôle | 897 | 5527 | 2318 |

| | | | |
|---|---|---|---|
| * α-CD = α-cyclodextrinepolysulfatée, β CD = β-cyclodextrinepolysulfatée, γ CD = γ-cyclodextrinepolysulfatée, CS =chondroitinesulfate, CPS = chondroïtinepolysulfate | | | |

Ce tableau montre l'efficacité remarquable des cyclodextrines polysulfatées, supérieure à celle des produits de référence, soit sur la production d'aggrécanes associés aux cellules, soit de ceux présents dans la matrice interterritoriale qui en est la conséquence.

Dans tous les cas, les résultats obtenus soit pour les produits de l'invention, soit pour les produits de référence, sont supérieurs à ceux relevés dans le groupe contrôle.

### EXEMPLE 2: Composition pharmaceutique

**Comprimés dosés à 100 mg de γ-cyclodextrinepolysulfatée, sel de sodium**

### Formule de préparation pour 1000 comprimés

| | |
|---|---|
| γ-cyclodextrinepolysulfatée, sel de sodium | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Utilisation d'une cyclodextrine polysulfatée ou d'un de ses sels d'addition comme seul ingredient actif pour l'obtention d'une composition pharmaceutique destinée au traitement de l'arthrose.

2. Utilisation selon la revendication 1, où la cyclodextrine polysulfatée est une γ-cyclodextrine polysulfatée.

3. Utilisation selon la revendication 1, où la cyclodextrine polysulfatée est une α-cyclodextrine polysulfatée.

4. Utilisation selon la revendication 1, où la cyclodextrine polysulfatée est une β-cyclodextrine polysulfatée.

5. Composition pharmaceutique pour l'utilisation dans le traitement de l'arthrose consistant essentiellement d'une cyclodextrine polysulfatée ou un de ses sels d'addition, en combinaison avec un ou plusieurs excipients, non toxiques, pharmaceutiquement acceptables.

6. Composition pharmaceutique selon la revendication 5, où la cyclodextrine polysulfatée est une γ-cyclodextrine polysulfatée.

7. Composition pharmaceutique selon la revendication 5, où la cyclodextrine polysulfatée est une α-cyclodextrine polysulfatée.

8. Composition pharmaceutique selon la revendication 5, où la cyclodextrine polysulfatée est une β-cyclodextrines polysulfatée.

9. Composition pharmaceutique selon une des revendications 5 à 9, où les excipients sont des diluants, des lubrifiants, des liants, des agents de désintégration, des absorbants, des colorants, ou des édulcorants.

10. Utilisation selon une des revendications 1 à 4, où la posologie de la cyclodextrine polysulfatée ou d'un de ses sels d'addition est comprise entre 50 mg et 1,500 mg par 24 heures.

11. Utilisation selon une des revendications 1 à 4 pour l'obtention d'une composition pharmaceutique sous forme de préparation injectable.

## Claims

1. Use of a polysulphated cyclodextrin or one of its addition salts as the only one active ingredient to obtain a pharmaceutical composition useful for the treatment of the arthrosis.

2. Use as claimed in claim 1, wherein the polysulphated cyclodextrin is a polysulphated γ-cyclodextrin.

3. Use as claimed in claim 1, wherein the polysulphated cyclodextrin is a polysulphated α-cyclodextrin.

4. Use as claimed in claim 1, wherein the polysulphated cyclodextrin is a polysulphated β-cyclodextrin.

5. A pharmaceutical composition useful for the treatment of the arthrosis consisting essentially of a polysulphated cyclodextrin or one of its addition salts, in combination with one or several non-toxic, pharmaceutically acceptable excipients.

6. A pharmaceutical composition as claimed in claim 5, wherein the polysulphated cyclodextrin is a polysulphated γ-cyclodextrin.

7. A pharmaceutical composition as claimed in claim 5, wherein the polysulphated cyclodextrin is a polysulphated α-cyclodextrin.

8. A pharmaceutical composition as claimed in claim 5, wherein the polysulphated cyclodextrin is a polysulphated β-cyclodextrin.

9. A pharmaceutical composition in accordance with one of claims 5 to 9, wherein the excipients are diluents, lubricants, binders, disintegration agents, absorbents, colouring agents, or sweeteners.

10. Use in accordance with one of claims 1 to 4, wherein the posology of the polysulphated cyclodextrine or one of its addition salts is ranging between 50 mg and 1,500 mg per 24 hours.

11. Use in accordance with one of claims 1 to 4 to obtain a pharmaceutical composition in the form of an injectable preparation.

## Patentansprüche

1. Verwendung eines polysulfatierten Cyclodextrins oder eines dessen Zusatzsalze als einziger aktiven Inhaltsstoff zur Gewinnung einer Arzneimittelzubereitung zur Behandlung von Arthrose.

2. Verwendung nach Anspruch 1, wobei das polysulfatierten Cyclodextrin ein polysulfatierten γ-Cyclodextrin ist.

3. Verwendung nach Anspruch 1, wobei das polysulfatierten Cyclodextrin ein polysulfatierten α-Cyclodextrin ist.

4. Verwendung nach Anspruch 1, wobei das polysulfatierten Cyclodextrin ein polysulfatierten β-Cyclodextrin ist.

5. Arzneimittelzubereitung zur Verwendung bei der Behandlung von Arthrose, wesentlich bestehend aus einem polysulfatierten Cyclodextrin oder einem dessen Zusatzsalze, in Verbindung mit einem oder mehreren untoxischen pharmazeutisch verträglichen Trägerstoffen.

6. Arzneimittelzubereitung nach Anspruch 5, wobei das polysulfatierten Cyclodextrin ein polysulfatierten γ-Cyclodextrin ist.

7. Arzneimittelzubereitung nach Anspruch 5, wobei das polysulfatierten Cyclodextrin ein polysulfatierten α-Cyclodextrin ist.

8. Arzneimittelzubereitung nach Anspruch 5, wobei das polysulfatierten Cyclodextrin ein polysulfatierten β-Cyclodextrin ist.

9. Arzneimittelzubereitung nach einem der Ansprüche 5 bis 9, wobei die Trägerstoffe Verdünnungsmittel, Gleitmittel, Bindemittel, Abbaumittel, Absorptionsmittel, Farbmittel oder Süßungsmittel sind.

10. Verwendung nach einem der Ansprüche 1 bis 4, wobei die Dosierung des polysulfatierten Cyclodextrin oder eines dessen Zusatzsalze von 50 mg bis 1500 mg pro 24 Stunden beträgt.

11. Verwendung nach einem der Ansprüche 1 bis 4 zur Gewinnung einer Arzneimittelzubereitung als injizierbares Präparat.
